Europäisches Patentamt

European Patent Office

Office européen des brevets

(19)

(11) Veröffentlichungsnummer : **0 000 159**
**B2**

(12) **NEUE EUROPÄISCHE PATENTSCHRIFT**

(45) Veröffentlichungstag der neuen Patentschrift :
19.12.84

(21) Anmeldenummer : 78100177.1

(22) Anmeldetag : 16.06.78

(51) Int. Cl.³ : **C 07 C 29/74**, C 07 C 31/20

(54) Verfahren zur Behandlung von Butandiol, das als Kondensat bei der Herstellung von Polybutylenterephthalaten erhalten wurde.

(30) Priorität : 24.06.77 DE 2728407

(43) Veröffentlichungstag der Anmeldung :
10.01.79 Patentblatt 79/01

(45) Bekanntmachung des Hinweises auf die Patenterteilung : 23.07.80 Patentblatt 80/15

(45) Bekanntmachung des Hinweises auf die Entscheidung über den Einspruch : 19.12.84 Patentblatt 84/51

(84) Benannte Vertragsstaaten :
BE DE FR GB NL

(56) Entgegenhaltungen :
BE-A-  720 958
FR-A- 1 081 681
NL-A- 7 002 510
Kirk-Othmer, Encyclopedia of Chemical Technology,
2. Auflage, 1965, Vol. 8, Seiten 356-357

(73) Patentinhaber : **BASF Aktiengesellschaft**
**Carl-Bosch-Strasse 38**
**D-6700 Ludwigshafen (DE)**

(72) Erfinder : **Beer, Ludwig, Dr.**
**Mundenhelmer Strasse 160**
**D-6700 Ludwigshafen (DE)**
Erfinder : **Mandel, Heinrich**
**Albrecht-Duerer-Ring 29a**
**D-6710 Frankenthal (DE)**

Jouve, 18, rue St-Denis, 75001 Paris, France

EP 0 000 159 B2

## Beschreibung

Gegenstand der Erfindung ist ein Verfahren zur Behandlung von Butandiol, das als Kondensat bei der Herstellung von Polybutylenterephthalaten erhalten wurde, mit alkalischen Mitteln.

Es ist bekannt, Polybutylenterephthalate herzustellen durch Umesterung von Terephthalsäuredimethylester mit Butandiol-1,4 oder direkte Veresterung von Terephthalsäure mit Butandiol-1,4 und das so erhaltene Bis-Hydroxybutylterephthalat und dessen Oligomere anschließend unter Abspaltung von Butandiol-1,4 bei höherer Temperatur unter vermindertem Druck zu polykondensieren (vgl. DT-OS 22 14 775 und 25 14 116). Bei der Polykondensation wird das durch Abspaltung entstehende Butandiol-1,4 sowie im Überschuß vorhandenes Butandiol-1,4 bei erhöhter Temperatur und unter vermindertem Druck abdestilliert. Aufgrund der sehr niedrigen Drücke, die bei der Polykondensation angewandt werden müssen, enthalten die abgehenden Brüden nicht nur Butandiol-1,4 sondern auch geringe Mengen an Bis-Hydroxybutylterephthalat und dessen Oligomere sowie katalysatorhaltige Bestandteile und Produkte mit unbekannter Zusammensetzung. Darüber hinaus kann auch Polybutylenterephthalat in feinen Tröpfchen mitgerissen werden. Die Brüden aus der Polykondensation werden kondensiert z. B. an Wärmetauscherflächen oder insbesondere mittels Einsprühkondensatoren, die den Vakuumaggregaten vorgeschaltet sind. Diese mit den Butandiol-Brüden abgehenden Begleitstoffe sind teilweise Festprodukte, die in kaltem oder mäßig warmem Butandiol nur wenig löslich sind. Infolge dessen scheiden sie sich bei der Kondensation der Brüden in den Kreisläufen der Einspritzkondensatoren als Feststoffe aus, die vor allem im kontinuierlichen, aber auch im diskontinuierlichen Betrieb erhebliche Störungen, z. B. durch Verstopfungen der Verteilerdüsen an den Einspritzkondensatoren oder Belegen der Kühlflächen mit ausgeschiedenen Feststoffen verursachen.

Ähnliche Probleme mit Feststoffausscheidungen im kondensierten Äthylenglykol bei der Herstellung von Polyäthylenterephthalat werden durch Zugabe von Alkalihydroxiden oder Alkalicarbonaten verhindert, wie in der US-PS 2,793,235 beschrieben wird. Um eine wirkungsvolle Auflösung der Polyester zu bewerkstelligen, müssen jedoch mindestens stöchiometrische Mengen an Alkalihydroxid angewandt werden. Die dabei entstehenden Verseifungsprodukte lösen sich im Äthylenglykol. Behandelt man in entsprechender Weise Butandiol-1,4, das bei der Herstellung von Polybutylenterephthalaten anfällt, so zeigt sich, daß auch nach mehrstündiger Einwirkungsdauer die ausgeschiedenen Feststoffe nicht in Lösung zu bringen sind.

Es war deshalb die technische Aufgabe gestellt, Butandiol, das als Kondensat bei der Herstellung von Polybutylenterephthalaten anfällt, so zu behandeln, daß keine Feststoffausscheidungen eintreten.

Diese Aufgabe wird gelöst in einem Verfahren zur Behandlung von Butandiol, das als Kondensat bei der Herstellung von Polybutylenterephthalaten anfällt, mit alkalischen Mitteln, wobei man dem Kondensat bei einer Temperatur von 40 °C bis 60 °C 0,01 bis 0,2 Gewichtsprozent, bezogen auf das Kondensat, Alkalialkoholate oder Alkalihydride zusetzt. Das neue Verfahren hat den Vorteil, daß die im Butandiolkondensat enthaltenden Feststoffe auf einfache Weise und wirkungsvoll in Lösung gebracht werden, so daß keine Schwierigkeiten in den Kondensatoren auftreten. Weiter hat das neue Verfahren den Vorteil, daß weniger als die stöchiometrische Menge an den erfindungsgemäßen alkalischen Mitteln benötigt werden. Dies ist insofern überraschend, als es sich bei den Alkalialkoholaten um Verbindungen handelt, die in ihrer chemischen Wirksamkeit häufig den Alkalihydroxiden entsprechen. Die zur vollständigen Auflösung der Feststoffe im Butandiol notwendige Menge an Alkalialkoholaten liegt um eine Größenordnung niedriger als die Natriumhydroxidmenge, die zur Auflösung von Feststoffen in Äthylenglykol, das aus der Herstellung von Polyäthylenterephthalat stammt, notwendig ist.

Das behandlungsbedürftige Butandiol-1,4 fällt als Kondensat bei der Herstellung von Polybutylenterephthalaten an. Hierbei geht man beispielsweise von Dimethylterephthalat aus, estert dieses mit Butandiol-1,4 in Gegenwart von Umesterungskatalysatoren, bei Temperaturen von 160 bis 230 °C um und kondensiert das so erhaltene Bis-Hydroxybutylterephthalat, gegebenenfalls mehrstufig bei Temperaturen von 230 bis 270 °C und Drücken bis zu 13.3 Pa. Es ist auch möglich, bis zu 40 Mol.%, bezogen auf Dimethylterephthalat und/oder Butandiol-1,4 andere Polyester bildende Ausgangsstoffe mitzuverwenden. Geeignet sind beispielsweise Dimethylester von Alkandicarbonsäuren mit 4 bis 12 Kohlenstoffatomen oder Isophthalsäure, ferner Alkandiole oder Cycloalkandiole mit bis zu 8 Kohlenstoffatomen. Geeignete Verfahren werden beispielsweise beschrieben in der DT-OS 25 14 116 oder 22 14 775. Bei der Herstellung von Polybutylenterephthalaten anfallende Kondensate enthalten neben Butandiol bis zu etwa 20 Gew.% Feststoffe. Daneben können, falls andere Diole als Ester bildende Komponenten mitverwendet werden, solche entsprechend ihrem Anteil z. B. bis zu 40 Gew.% im Kondensat enthalten sein. Behandlungsbedürftige Butandiolkondensate fallen im wesentlichen in der Vor- und Endkondensationsstufe an. Ein typisches Kondensat, das bei der Herstellung von Polybutylenterephthalat erhalten wird, enthält im wesentlichen Butandiol-1,4 und hat einen Feststoffgehalt von 3 bis 6 Gew.%. Der bei Raumtemperatur unlösliche Feststoffanteil beträgt etwa 1 bis 3 Gew.%.

Die Behandlung erfolgt durch Zusatz von Alkalialkoholaten oder Alkalihydriden. Besonders bevorzugt werden Alkalialkoholate, die sich von Alkanolen oder Cycloalkanolen mit bis zu 10 Kohlenstoffatomen, insbesondere mit bis zu 7 Kohlenstoffatomen oder Alkandiolen mit 2 bis 6 Kohlenstoffatomen ableitet. Besondere technische Bedeutung haben Natriumalkoholate, die sich von Alkanolen mit 1 bis 4 Kohlenstoffatomen ableiten, erlangt. Geeignete Verbindungen sind beispielsweise Natriummmethylat, Natriumäthylat oder Natriumbutylat.

Man setzt dem zu behandelnden Kondensat Alkalialkoholate oder Alkalihydride in Mengen von 0,01 bis 0,2 Gew.%, vorzugsweise 0,01 bis 0,1 Gew.% zu. Wählt man die Menge zu niedrig, so dauert der Lösevorgang zu lange, wählt man sie jedoch zu hoch, so können sich Schwierigkeiten durch alkalihaltige Feststoffabscheidungen bei der destillativen Wiederaufbereitung des Butandiols ergeben. Die optimale Zugabemenge an Alkalialkoholat kann einfach durch laufende Kontrolle des pH-Wertes des zu behandelnden Kondensats reguliert werden. Vorteilhaft soll der pH-Wert über 9, vorzugsweise von 10 bis 12 betragen.

Die Alkalialkoholate werden im allgemeinen in konzentrierter alkoholischer Lösung eingesetzt. Besonders gut bewährt hat sich eine methanolische Natriummethylatlösung. Es ist aber auch möglich, festes Alkalialkoholat in Butandiol zu lösen und diese Lösung zur Behandlung zu verwenden.

Um eine möglichst rasche Auflösung der unlöslichen Festbestandteile in Butandiol zu ermöglichen, führt man die Behandlung bei einer Temperatur von 40 °C bis 60 °C, insbesondere 40 bis 50 °C durch. Diese Temperatur läßt einen Druck von 66,6 bis 266,6 Pa ohne Schwierigkeiten zu.

Die erfindungsgemäße Arbeitsweise zur Behandlung von Butandiol eignet sich besonders gut für alle Polybutylenterephthalatverfahren, bei denen das während der Polykondensation abdestillierende Butandiol in geeigneten Einspritzkondensatoren kondensiert wird, die mit einem Butandiolkreislauf versehen sind. Bei kontinuierlicher Herstellung ist es vorteilhaft, die Alkalialkoholate als alkoholische Lösung kontinuierlich dem Butandiolkreislauf zuzumischen. Bei diskontinuierlicher Arbeitsweise bietet es sich auch an, die Alkalialkoholatlösung portionsweise zuzugeben. Es versteht sich, daß man die angegebenen Konzentrationen an Alkalialkoholat oder Alkalihydrid während der gesamten Arbeitsdauer, aufrecht erhält.

Vorteilhaft unterstützt man die Auflösung der festen Anteile durch Rühren oder Umpumpen.

Es hat sich herausgestellt, daß durch den Zusatz von Alkalialkoholaten oder Alkalihydriden in den beaufschlagten Vorrichtungen die Korrosionsneigung praktisch völlig beseitigt wird. Ferner hat es sich erwiesen, daß man bei der destillativen Aufarbeitung reineres Butandiol als bisher zurückgewinnt. Das Butandiol kann wieder für die Herstellung von Polybutylenterephthalaten verwendet werden.

Das Verfahren nach der Erfindung sei an folgenden Beispielen veranschaulicht. Die angegebenen Teile sind Gewichtsteile. Sie verhalten sich zu den Raumteilen wie Kilogramm zu Liter. Die angegebenen Prozente sind Gewichtsprozente.

Beispiel 1

Man geht von Butandiol aus, das bei der Herstellung von Polybutylenterephthalat als Kondensat erhalten wurde und einen Gesamtfeststoffgehalt von 5 % hat. Der bei Raumtemperatur unlösliche Feststoffanteil beträgt 2 %. 1 000 Teile Butandiol der genannten Qualität werden unter Rühren bei 55 bis 60 °C mit 1,7 Teilen einer 30 %-igen methanolischen Natriummethylatlösung (entsprechend 0,05 Gew.% Na-methylat bzw. Butandiol) versetzt und unter Rühren im angegebenen Temperaturbereich weiter behandelt. Nach 2 Stunden Reaktionszeit sind die Festbestandteile bis auf eine geringfügige Opaleszenz völlig in Lösung gegangen. Der mittels Wasserstoffelektrode gemessene pH-Wert der Lösung liegt bei 11. Die so erhaltene Lösung zeigt nach eintägigem Stehen bei Raumtemperatur keine Feststoffabscheidungen mehr und ist damit für die anschließende destillative Weideraufarbeitung genügene stabil.

Beispiel 2

Man verfährt wie in Beispiel 1 beschrieben, verwendet jedoch anstatt Natriummethylat 6 Teile einer 10 %-igen Lösung von Natriumäthylat in Butandiol (entsprechend 0,06 Gew.%). Nach 2 stündigem Rühren ist der unlösliche Feststoffanteil praktisch vollständig in Lösung gegangen. Der pH-Wert der Lösung liegt bei 11.

Vergleichsversuch 1

Man verwendet 1 000 Teile Butandiol wie in Beispiel 1 beschrieben und versetzt dieses unter Rühren bei 55 bis 60 °C mit 8,6 Teilen einer 50 %-igen wäßrigen Natronlauge (entsprechend 0,43 Gew.% NaOH). Nach 2 stündigem Rühren im angegebenen Temperaturbereich zeigt das Butandiol eine unverändert starke Trübung durch Anwesenheit ungelöster Feststoffteilchen. Bereits nach 1 stündigem Stehen bei Raumtemperatur scheiden sich am Gefäßboden größere Mengen an unlöslichem Feststoff ab.

Vergleichsversuch 2

Man verfährt wie im Vergleichsversuch 1 beschrieben, wendet jedoch Temperaturen von 65 bis 70 °C an und erhöht die Zugabe an Natronlauge auf 26 Teile 50 gew.%-ige wäßrige Natronlauge (entsprechend 1,3 Gew.% Natriumhydroxid). Nach 2 stündigem Rühren bei der angegebenen Temperatur hat sich der unlösliche Feststoffanteil praktisch nicht vermindert. Nach kurzem Stehen bei Raumtemperatur setzt sich eine erhebliche Menge an Feststoff ab.

Vergleichsversuch 3

Man behandelt Butandiol entsprechend Vergleichsversuch 1 mit 60 Teilen einer 20 %-igen wäßrigen Natriumcarbonatlösung (entsprechend 1,2 % Natriumcarbonat). Nach 2 stündigem Rühren ist keine Auflösung der Festbestandteile festzustellen.

Vergleichsversuch 4

Bei der kontinuierlichen Herstellung von Polybutylenterephthalat fallen stündlich in der Vor- und Endkondensationsstufe 18 kg Butandiol als Kondensat an. Dieses enthält im stationären Betrieb etwa 5 % Feststoffe. Bei Raumtemperatur beträgt der Anteil an unlöslichen Feststoffen etwa 2 %. Die Butandiolkondensate as Vor- und Endkondensation werden in einem Kreislauf vereinigt, der mit den zur Brüdenabscheidung verwendeten Einspritzkondensatoren der Vor- und Endkondensationsstufe verbunden ist. Das überschüssige Butandiol wird in regelmäßigen Abständen aus einem im Kreislauf befindlichen Puffergefäß abgezogen. Dieser Kreislauf wird zur Aufrechterhaltung eines fließfähigen Gemisches von Zeit zu Zeit mit Frischbutandiol verdünnt. Trotz dieser Maßnahme sind nach einigen Tagen Betriebszeit mehrere der am Kondensator vorhandenen Einspritzdüsen verstopft, so daß sie mechanisch gereinigt werden müssen. Außerdem steigt der Düsenvordruck laufend an und die Kühlleistung der im Butandiolkreislauf befindlichen Kühler nimmt durch Belegen der Kühlflächen mit Feststoffen merklich ab. Nach 4 Wochen Betriebszeit müssen die Leitungen mechanisch gereinigt und der Kühler zur Reinigung gewechselt werden.

Vergleichsversuch 5

Man verfährt wie im Vergleichsversuch 4 beschrieben, leitet jedoch in eine turbulent durchströmte Leitung des Butandiolkreislaufs unter Einhaltung einer Temperatur von 45 bis 50 °C kontinuierlich und stündlich 156 Teile einer 50 %-igen wäßrigen Natronlauge (entsprechend 0,43 % NaOH bez. Butandiol-An-fall) ein. Nach einigen Tagen Betriebszeit ist das Butandiol im Kreislauf stark mit Feststoffen durchsetzt. Die Verstopfung der Einspritzdüsen ist unverändert stark. Der Düsenvordruck in Einspritzkondensatoren und die Kühleistung des Kreislaufkühlers verschlechtern sich fortlaufend.

Beispiel 3

Man verfährt wie in Vergleichsversuch 5, setzt dem Butandiolkreislauf jedoch kontinuierlich und je Stunde 35 g einer 30 %-igen methanolischen Natriummethylatlösung (entsprechend 0,06 % Natriummethylat bez. Butandiol) zu. Das Butandiol im Kreislauf hält man auf einer Temperatur von 45 bis 50 °C. Auf eine periodische Verdünnung des Kreislaufs mit frischem Butandiol wird verzichtet. Beim Betrieb der Anlage ist keine Feststoffausscheidung festzustellen und während einer 3 monatigen ununterbrochenen Betriebsdauer treten keinerlei Betriebsstörungen durch Feststoffabscheidungen im Butandiolkreislauf ein.

**Ansprüche**

1. Verfahren zur Behandlung von Butandiol, das als Kondensat bei der Herstellung von Polybutylenterephthalaten anfällt, mit alkalischen Mitteln, dadurch gekennzeichnet, daß man dem Kondensat bei einer Temperatur von 40 °C bis 60 °C 0,01 bis 0,2 Gew.%, bezogen auf das Kondensat, Alkalialkoholate oder Alkalihydride zusetzt.

2. Verfahren nach Anspruch 1, dadurch gekennzeichnet, daß man einen pH-Wert von größer als 9 aufrechterhält.

3. Verfahren nach den Ansprüchen 1 und 2, dadurch gekennzeichnet, daß man Natriumalkoholate, die sich von Alkanolen mit 1 bis 4 Kohlenstoffatomen ableiten, anwendet.

4. Verfahren nach den Ansprüchen 1 bis 3, dadurch gekennzeichnet, daß man Natriummethylat verwendet.

**Claims**

1. A process for treating butanediol, obtained as a condensate in the production of polybutylene terephthalates, with alkaline agents, characterized in that 0.01 to 0.2 % by weight, based on the condensate, of an alkali metal alcoholate or an alkali metal hydride is added to the condensate at a temperature of from 40 °C to 60 °C.

2. A process according to claim 1, characterized in that a pH or more than 9 is maintained.

3. A process according to claims 1 and 2, characterized in that sodium alcoholates derived from alkanols of 1 to 4 carbon atoms are used.

4. A process according to claims 1 to 3, characterized in that sodium methylate is used.

**Revendications**

1. Procédé de traitement du butane-diol obtenu comme condensat dans la préparation de poly(téréphtalates de butylène) avec des agents alcalins, caractérisé en ce que l'on ajoute au condensat, à une température de 40 à 60 °C, entre 0,01 et 0,2 % de son poids d'alcoolates ou d'hydrures alcalins.

2. Procédé suivant la revendication 1, caractérisé en ce que l'on maintient le pH à une valeur supérieure à 9.

3. Procédé suivant la revendication 1 ou la revendication 2, caractérisé en ce que l'on emploie des alcoolates de sodium dérivés d'alcanols en $C_1$ à $C_4$.

4. Procédé suivant les revendications 1 à 3, caractérisé en ce que l'on emploie du méthylate de sodium.